# EUROPEAN PATENT SPECIFICATION

(11) **EP 4 301 420 B1**
(45) Date of publication and mention of the grant of the patent: **07.05.2025**
(21) Application number: 22714773.3
(22) Date of filing: 01.03.2022
(51) Int. Cl.: A61L 2/08, A61B 90/70, A61M 25/00, C01B 32/00

(54) **REUSABLE LIGHT-ACTIVATED STERILIZATION SYSTEM FOR MEDICAL DEVICES**
WIEDERVERWENDBARES LICHTAKTIVIERTES STERILISATIONSSYSTEM FÜR MEDIZINISCHE VORRICHTUNGEN
SYSTÈME DE STÉRILISATION ACTIVÉ PAR LA LUMIÈRE RÉUTILISABLE POUR DISPOSITIFS MÉDICAUX

(30) Priority: 01.03.2021 PT 2021007964
(43) Date of publication of application: 10.01.2024
(73) Proprietor: INEB-Instituto Nacional De Engenharia Biomédica, 4200-135 Porto (PT); Universidade Do Porto, 4099-002 Porto (PT)
(72) Inventor: DE CASTRO GONÇALVES DE ALMADA LOBO, Inês, 4150-249 Porto (PT); CARVALHO HENRIQUES, Ana Patrícia, 4200-384 Porto (PT); MOREIRA PINTO, Artur Daniel, 4200-465 Porto (PT); DE MAGALHAES, Fernao Domingos de Montenegro Baptista Malheiro, 4200-465 Porto (PT)
(74) Representative: Hoffmann Eitle
(86) International application number: PCT/EP2022/055057
(87) International publication number: WO 2022/184670

(56) References cited:
- JP-A- 2020 081 525
- US-A1- 2018 369 560
- HUI LIWEI ET AL: "Surface Disinfection Enabled by a Layer-by-Layer Thin Film of Polyelectrolyte-Stabilized Reduced Graphene Oxide upon Solar Near-Infrared Irradiation", APPLIED MATERIALS & INTERFACES, vol. 7, no. 19, 7 May 2015 (2015-05-07), US, pages 10511 - 10517, XP055924898, ISSN: 1944-8244, Retrieved from the Internet <URL:https://pubs.acs.org/doi/pdf/10.1021/acsami.5b02008> [retrieved on 20220524], DOI: 10.1021/acsami.5b02008

## Description

### Technical field

The present invention relates to a reusable integrated light-activated sterilization system. Further aspects of the present invention include a medical device including the reusable light-activated sterilization system, and more particularly a catheter cap including the reusable light-activated sterilization system.

### Background art

Bacterial adhesion to surfaces is the onset of biofilm formation and a hard problem to tackle, aggravated by the rise in drug-resistant bacteria, responsible for more than 500000 deaths globally/year. Prevention of bacterial adhesion and consequent biofilm formation is a demanding and costly task. It is, however, of utmost importance in different contexts directly related to human health and life. Several problems may arise when these issues are not efficiently treated, including microbial-induced corrosion, contamination of drinking water systems, oral diseases, failure of biomedical implants, and several other problems directly related to the public health. Endowing surfaces with antibacterial properties has been a widely explored strategy. Conventional solutions have focused on the use of biocidal agents, as antimicrobial nanoparticles or antibiotics, with the latter still being the major weapon to fight bacterial infections. However, antibiotics overuse has contributed to the development of bacterial resistance, one of the biggest threats to public health today. Therefore, alternative strategies are needed to successfully tackle the problem. Photoactive bactericidal surfaces killing bacteria through the use of light combined with an appropriate photoactive agent rather than with biocides have attracted particular attention. Antimicrobial photothermal therapy (PTT) and photodynamic therapy (PDT) are two promising light-based methods. PTT is based on the irradiation of a photothermal agent that converts light into heat, leading to a local temperature increase. As a consequence, membrane permeability increases, expression of heat shock proteins is upregulated, and cellular components denaturation occurs, eventually leading to bacterial death. In PDT, irradiation and excitation of a nontoxic photosensitizer starts the process of energy or electron transference to tissue oxygen molecules, inducing the production of singlet oxygen (¹O₂) and/or other reactive oxygen species (ROS), respectively. This leads to oxidation of different biological molecules, ultimately leading to bacterial death. Contrarily to antibiotics, the development of resistance by bacterial cells is highly unlikely when using PTT or PDT.

Normally, photoactive bactericidal surfaces make use of different molecules, including gold nanomaterials, metal nanoparticles, inorganic nanomaterials, and 2D materials, such as graphene-based materials (GBMs), as well as of combination of these nanomaterials to achieve complementary performances. GBMs show great potential to be used in these light-based therapies, due to their high photothermal conversion efficiency associated with remarkable broad-band optical absorption ability that arises from the closely spaced energy levels of the loosely held π electrons.

Current state-of-the-art on GBMs systems are however generally complex, with GBMs being combined with other materials or being functionalized with organic molecules or serving as immobilization platforms for metallic nanoparticles, antibiotics, or other photosensitizers. Moreover, these solutions generally rely on stimulation with lasers that are expensive, have a small irradiation area, and damage the surrounding tissues, commonly surpassing the irradiance limit of skin tolerance set by American National Standards Institute - ANSI (~0.33 W/cm² at 808 nm). Some examples are the combination of GO with red phosphorous, PHA polymer (polyhydroxyalkanoates), CuO nanowires, upconverted nanoparticles on TiO2, or the functionalization with Aldehydes or other organic materials. These systems very often rely on the PTT effect only or mainly on it. Efficient elimination of bacteria is generally obtained when temperature reaches over 100 ºC, as in the case of GO/PHA layer-by-layer film upon irradiation for 15 min by NIR (1064 nm, 0.085 W) laser. This in turn means that a significative temperature increase is to be obtained to have an efficient antibacterial effect. These drawbacks compromise their translation to clinical or real-life applications, with most reported works studying GBMs as suspensions in water rather than deposited on surfaces. Systems comprising graphene based layers and light sources are known from HUI LIWEI ET AL: "Surface Disinfection Enabled by a Layer-by-Layer Thin Film of Polyelectrolyte-Stabilized Reduced Graphene Oxide upon Solar Near-Infrared Irradiation" and JP 2020 081525 A.

### Technical problem

Therefore, a need remains for a sterilization system capable of delivering an efficient antibacterial effect while being safe for human use. Furthermore, there is a demand for a sterilization system that can be reused and that can be efficiently integrated in a medical device.

### Summary of the invention

The present invention is aimed at solving the problems of the prior art by providing a reusable light-activated sterilization system according to claim 1.

It has been found that a sterilization system comprising a material having layers of graphene and/or graphene oxide and a light emitting source (e.g a LED source) of near infrared as defined herewith results in an efficient sterilization system. In these sterilization systems, the layers of graphene and/or graphene oxide act as NIR-activated bactericidal platforms (photoactive surface) when irradiated by safe low-power NIR light emitting source (e.g. light emitting diodes (LEDs)).

Within the present disclosure with "Sterilization system" is meant a system having an antibacterial effect.

The sterilization systems according to the invention, advantageously delivers an antibacterial effect on both planktonic bacteria and on adherent bacteria thanks to the combination of the NIR light source as defined above with the layers of graphene and/or graphene oxide.

Where the present description refers to "preferred" embodiments/features, combinations of these preferred embodiments/features shall also be deemed as disclosed as long as this combination is technically meaningful.

Hereinafter, the use of the term "comprising" should be understood as disclosing in a non-limited way, that is to say that additional components or steps can be present or implemented, as long as this is technically meaningful. For a more restricted embodiment, the terms "consisting of" will be used and have to be understood as disclosing in a limited way, that is to say without any additional component or step.

### Brief description of the figures

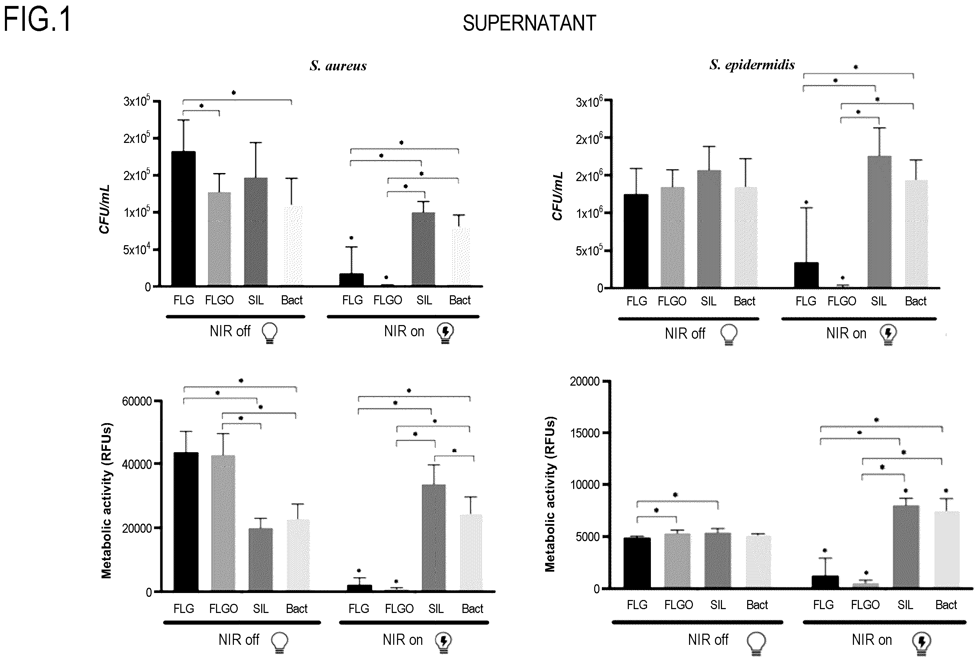

**Figure 1****:** Antibacterial performance of the sterilization systems according to the invention against planktonic bacteria (*S*. *aureus* and *S*. *epidermidis),* silicone films are used as a comparison. FLG = few-layer graphene film, FLGO = few-layer graphene oxide film, SIL = silicone film, Bact= Bacteria cultured in the absence of any material. 3 replicates of each material in each independent assay (n = 3).

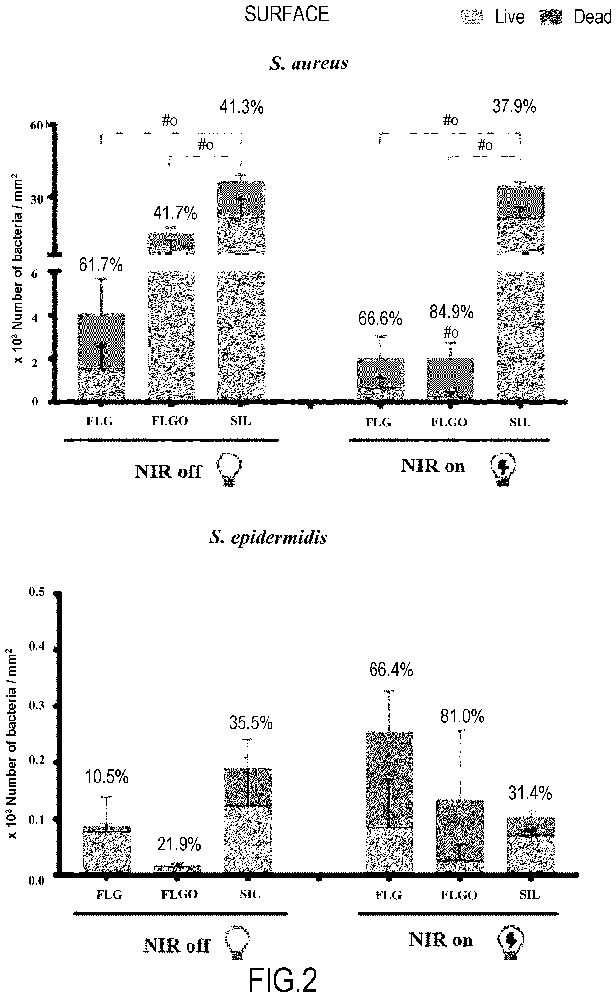

**Figure 2****:** Antibacterial performance of the sterilization systems according to the invention against adherent bacteria (*S*. *aureus* and *S*. *epidermidis).* The numbers on top of the columns represent the percentage of dead bacteria. Silicone films were used as a control material. #, o - statistically significant differences between samples regarding total number (#) and dead (o) adherent bacteria: one-way ANOVA to compare films within the same condition (either NIR off or NIR on) and unpaired t-test with Welch's correction to compare the same film between NIR off or NIR on conditions. FLG = few-layer graphene film, FLGO = few-layer graphene oxide film, SIL = silicone film. 3 replicates of each material in each independent assay (n=3).

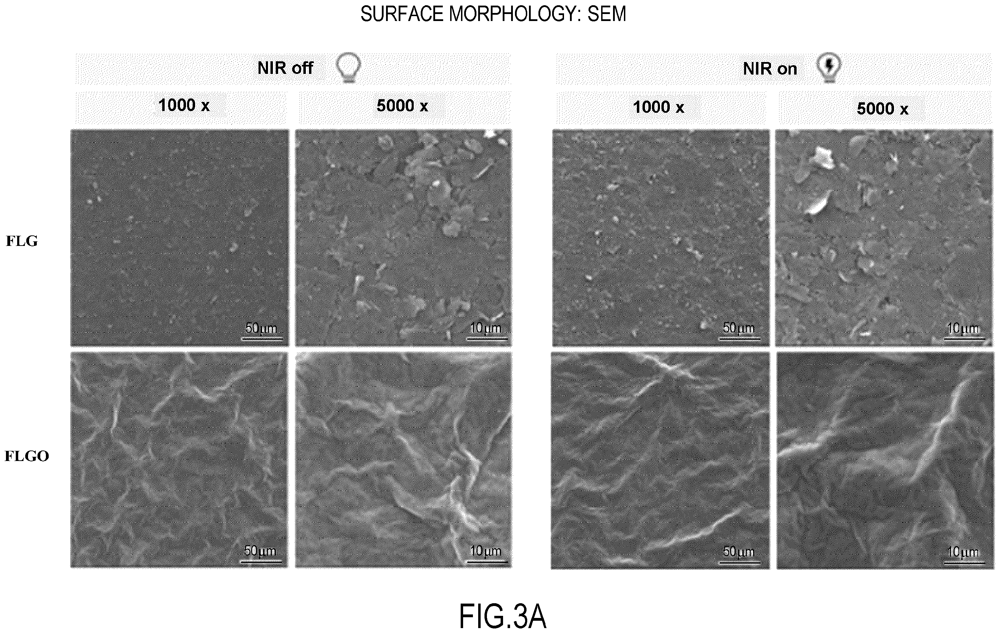

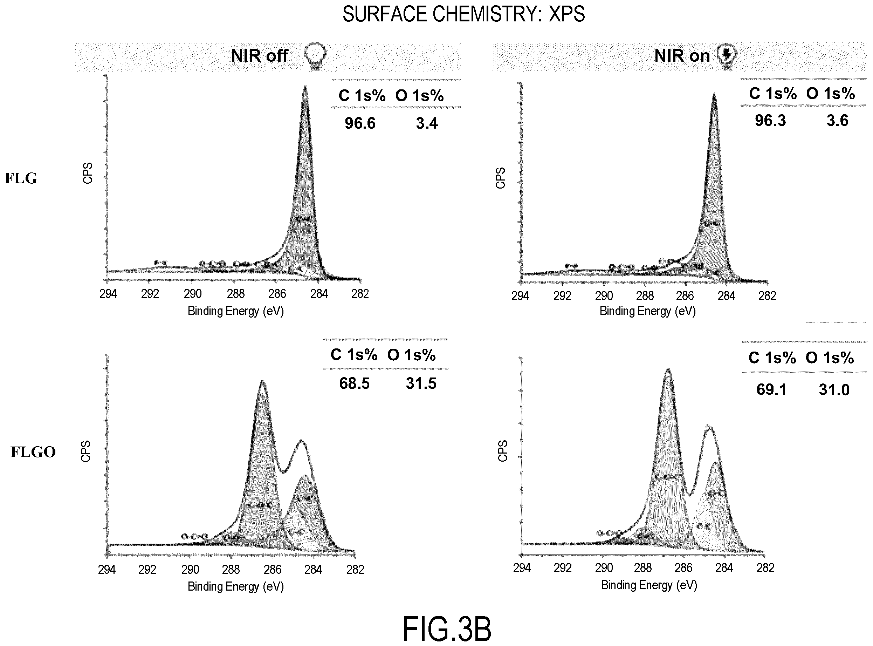

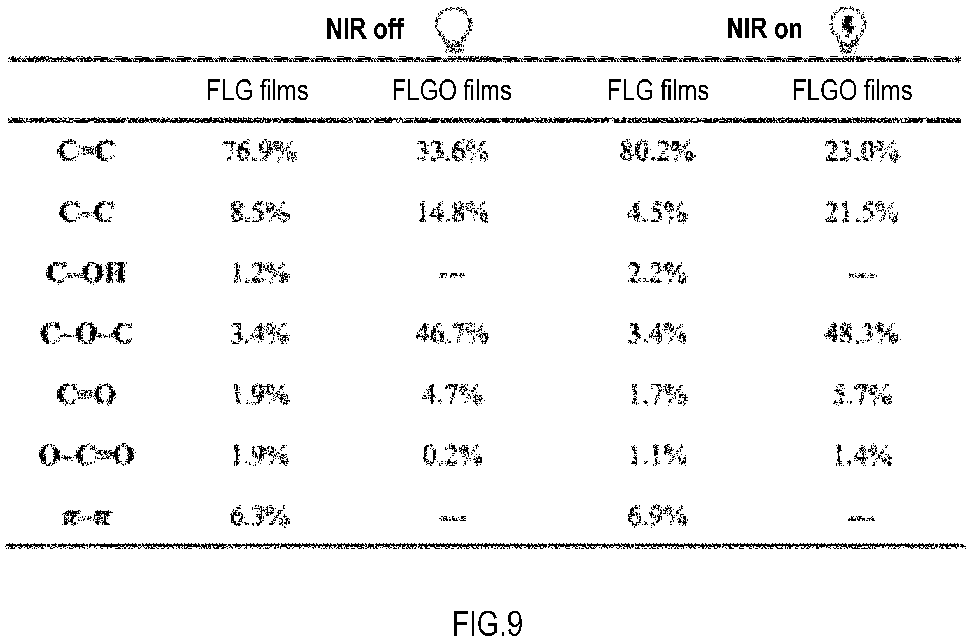

**Figure 3****:** Physicochemical features of FLG and FLGO films. (A) surface morphology and (B) chemistry of the materials having layers of graphene and/or graphene oxide according to the Examples. Morphology and topography obtained by SEM (1000x and 5000x magnifications, scale bar = 50 µm and 10 µm, respectively). Atomic percentages of carbon (C 1s) and oxygen (O 1s) extracted from XPS survey spectra. Chemical group percentages are shown in Figure 9. FLG = few-layer graphene film, FLGO = few-layer graphene oxide film.

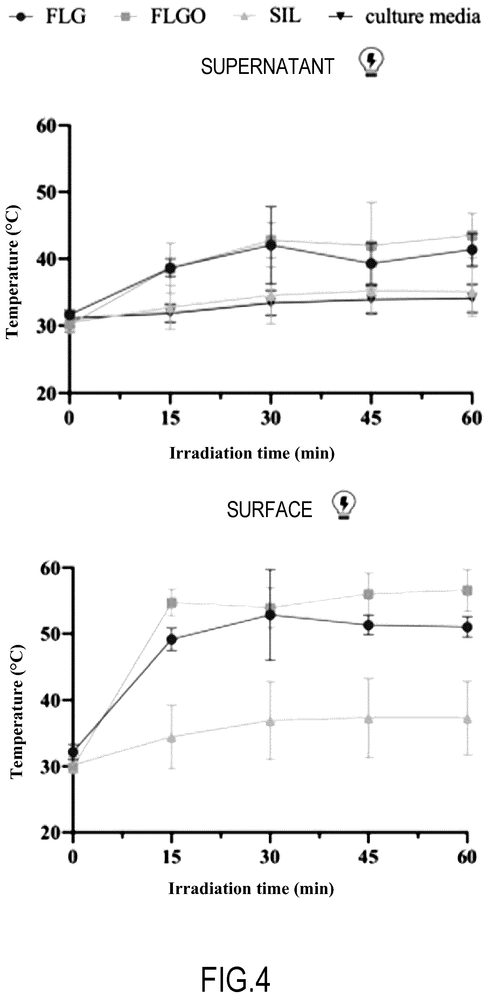

**Figure 4****:** Photothermal performance of FLG and FLGO films after 2 h incubation with culture media (TSB) followed by irradiation (NIR on) for 60 min (812 nm, 0.150 W/cm²). NIR-induced temperature change was assessed on films, surrounding supernatant and surface using a type K thermocouple microprobe. FLG = few-layer graphene film, FLGO = few-layer graphene oxide film, SIL = silicone film.

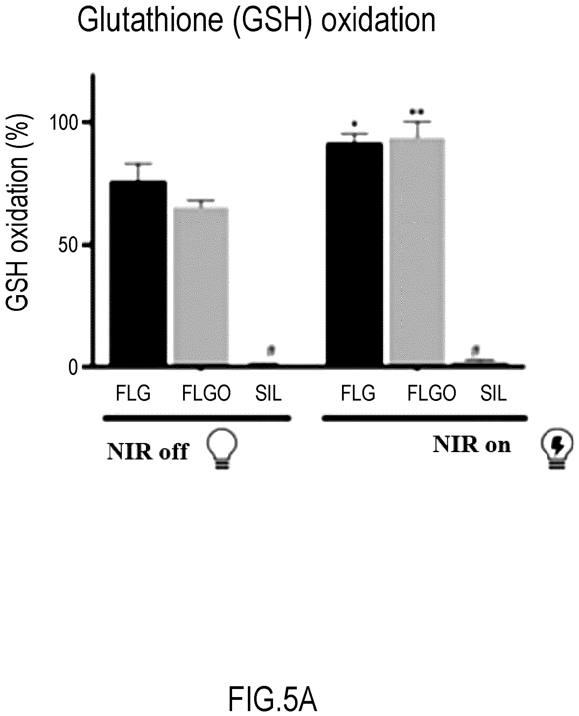

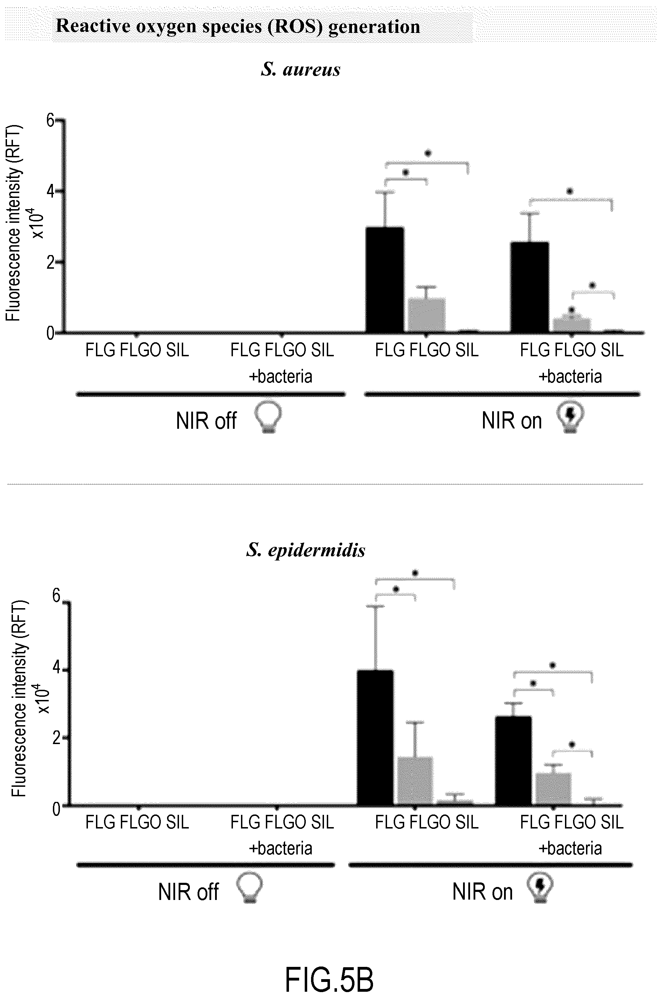

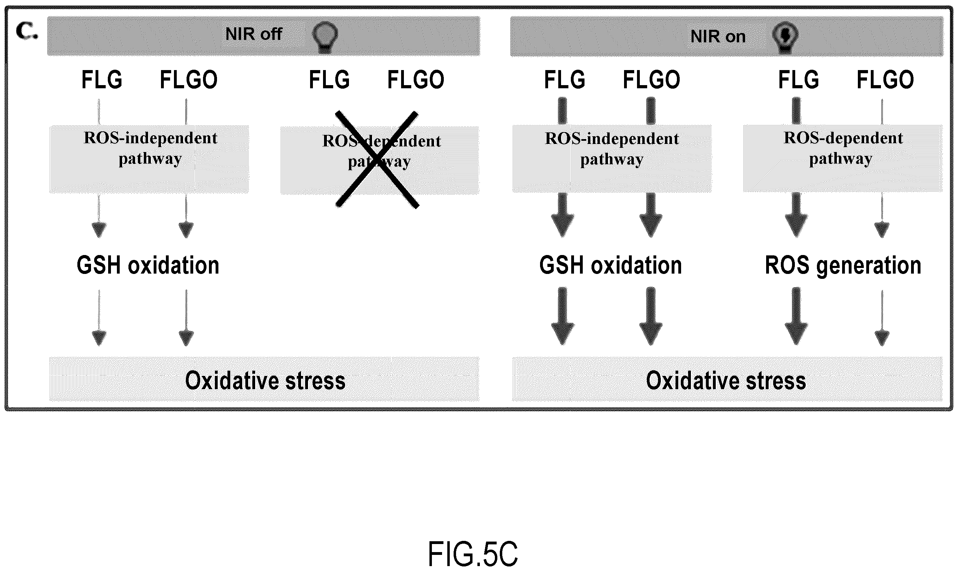

**Figure 5****:** Photodynamic properties of FLG and FLGO films. (A) determination of glutathione (GSH) depletion by Ellman's reagent and (B) reactive oxygen species (ROS) generation by FLG/FLGO films and by FLG/FLGO films in contact with bacteria (*S*. *aureus* and *S*. *epidermidis)* using DCFH-DA probe, when samples were kept in the dark (NIR off) or irradiated (NIR on) for 45 min (812 nm, 0.150 W/cm²). (C) Scheme summarizing the putative pathways for occurrence of oxidative stress. * Statistical significance, p < 0.05: one-way ANOVA to compare films within the same condition (either NIR off or NIR on) and unpaired t-test with Welch's correction to compare the same film between NIR off or NIR on conditions. * alone indicates significant differences from its counterpart in NIR off condition. FLG = few-layer graphene film, FLGO = few-layer graphene oxide film, SIL = silicone film.

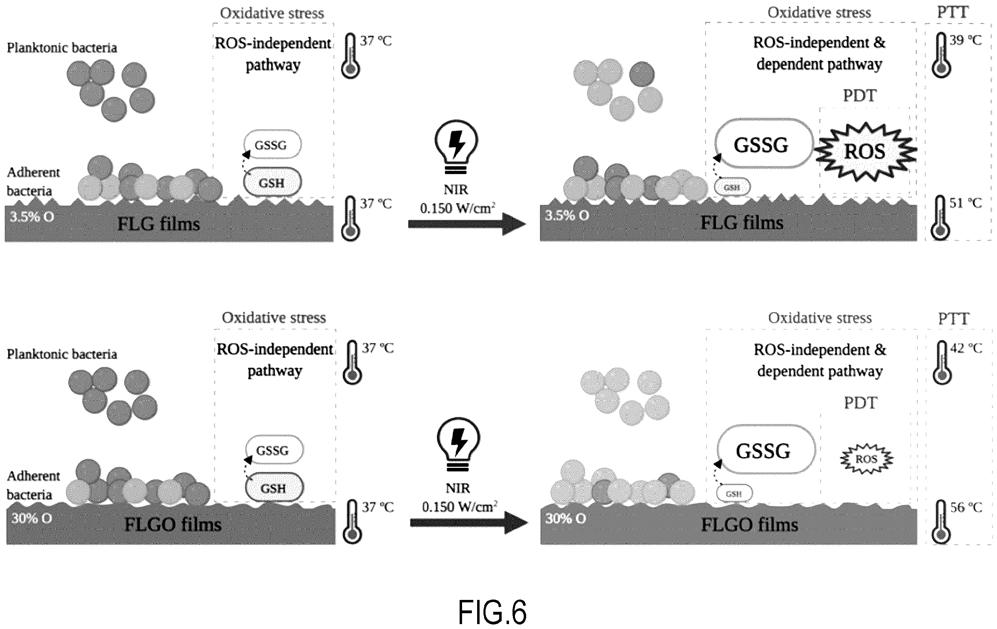

**Figure 6****:** Mechanism of action of FLG and FLGO films towards bacteria when kept in the dark (NIR off, left) and upon irradiation (NIR on, right). GSH = glutathione, GSSG = glutathione, disulfide (oxidized glutathione), ROS = reactive oxygen species, PDT = photodynamic therapy, PTT = photothermal therapy, NIR = near-infrared, FLG = few-layer graphene film, FLGO = few-layer graphene oxide film. Light grey in the representation are dead bacteria, dark grey live bacteria.

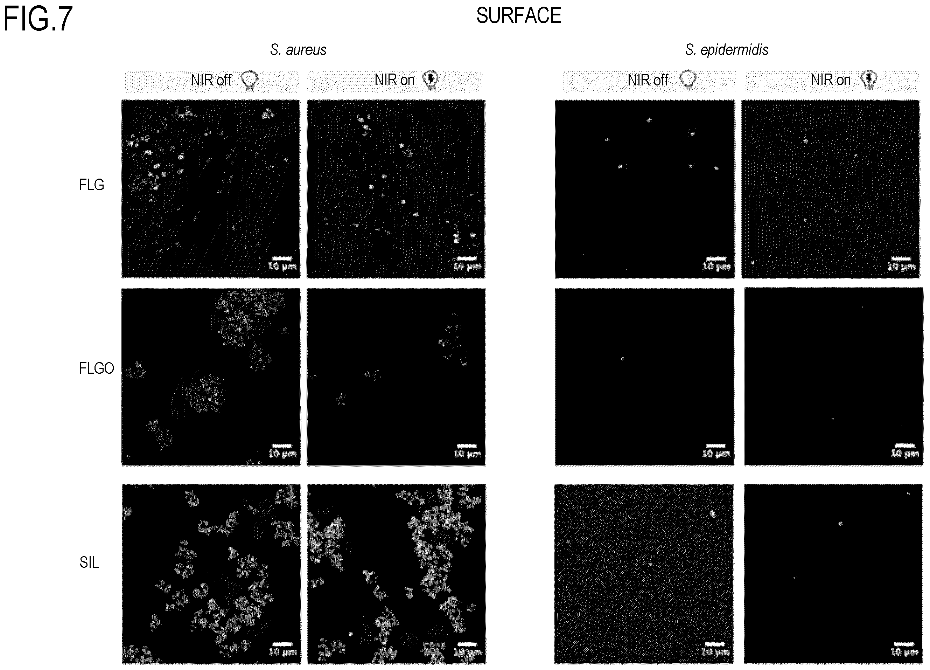

**Figure 7****:** Adherent bacteria on FLG, FLGO and SIL films. After 2 h incubation with *S*. *aureus* and *S*. *epidermidis,* FLG and FLGO films were either kept in the dark (NIR off) or irradiated (NIR on) for 45 min (812 nm, 0.150 W/cm²). Adherent bacteria were stained with SYTO9 (live) (grey spots) or PI (dead) (white spots) and images were obtained by IN Cell Analyzer 2000 (scale bar = 10 µm).

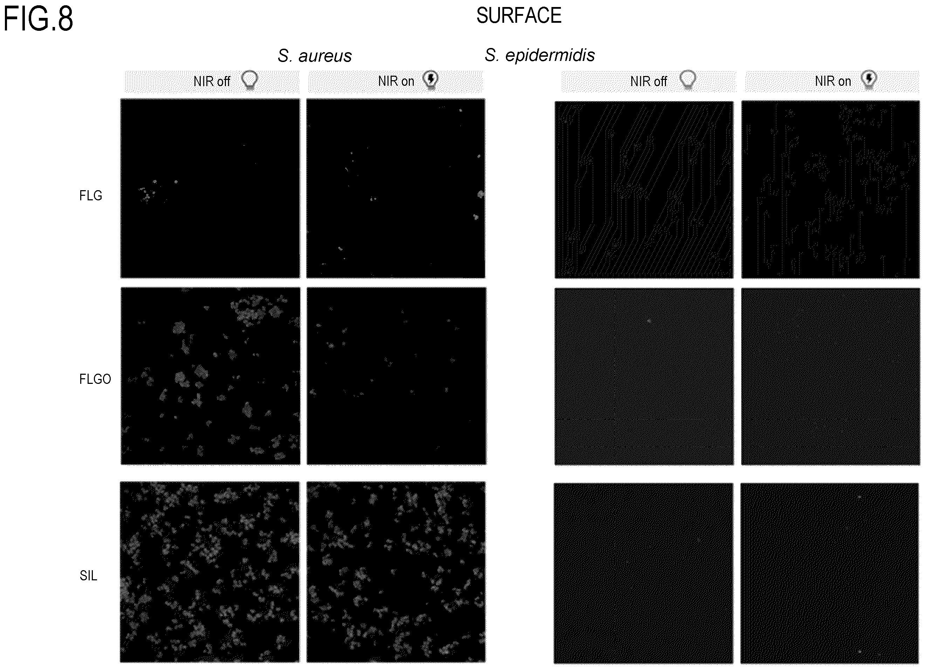

**Figure 8****:** Zoom out of adherent bacteria on FLG, FLGO and SIL films. After 2 h incubation with *S*. *aureus* and *S*. *epidermidis,* FLG and FLGO films were either kept in the dark (NIR off) or irradiated (NIR on) for 45 min (812 nm, 0.150 W/cm²). Adherent bacteria were stained with SYTO9 (live) (grey spots) or PI (dead) (white spots) and images were obtained by IN Cell Analyzer 2000 (scale bar = 10 µm).
**Figure 9****:** Chemical composition of the surfaces when kept in the dark (NIR off) or irradiated (NIR on) for 45 min (Examples 1-2, Comparative Examples 1-2). Exposed functional groups percentage extracted from XPS survey spectra: double carbon bonds (C=C), simple carbon bonds (C-C), hydroxyl groups (C-OH), epoxy groups (C-O-C), carbonyl groups (C=O), carboxyl groups (O-C=O), and pi-pi bonds (π-π).

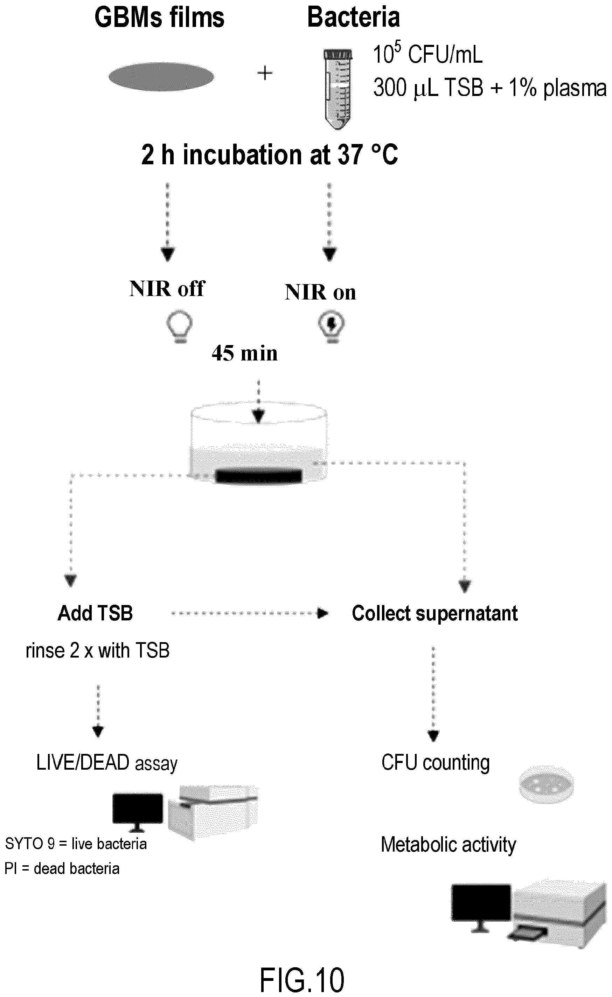

**Figure 10****:** Schematic representation of the antimicrobial assay procedure (Examples 1-2, Comparative Examples 1-3). After 2 h incubation of FLG and FLGO films with *S*. *aureus* or *S*. *epidermidis* to allow bacterial adhesion, samples were either kept in the dark (NIR off) or irradiated (NIR on, 812 nm, 0.150 W/cm²) for 45 min. Viability of adherent and planktonic bacteria were analysed by live/dead fluorescent assay, and colony forming unit (CFU) and metabolic activity assays, respectively. The image representing the NIR light source is not indicative of the positioning of the light source. In the Examples the NIR light source was placed below the plastic well comprising the films.

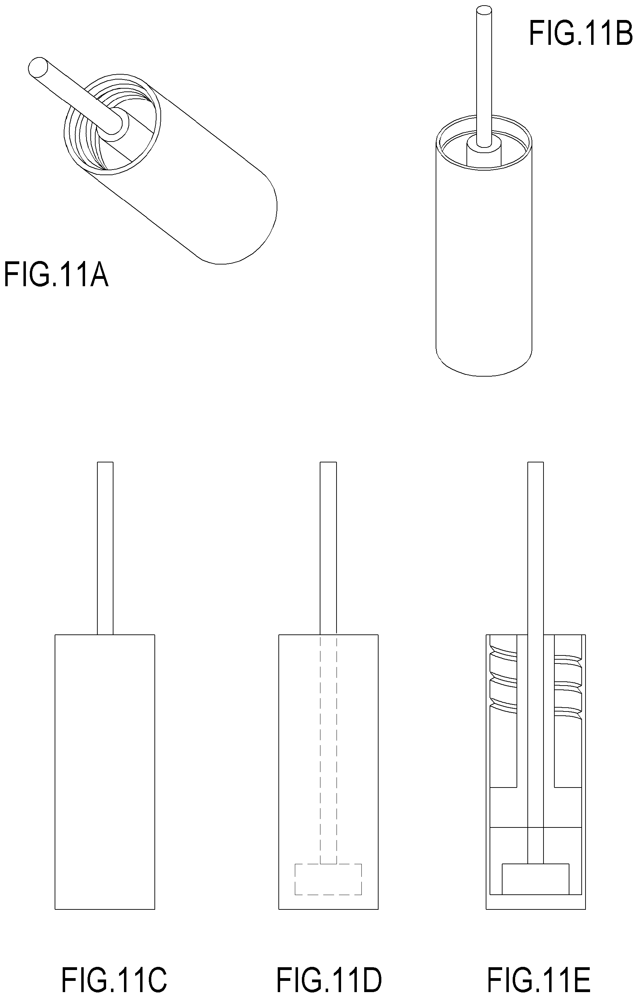

**Figure 11 A-E**: Schematic representation of one embodiment of the Sterilization System, *i.e*. the sterilization cap for a catheter.

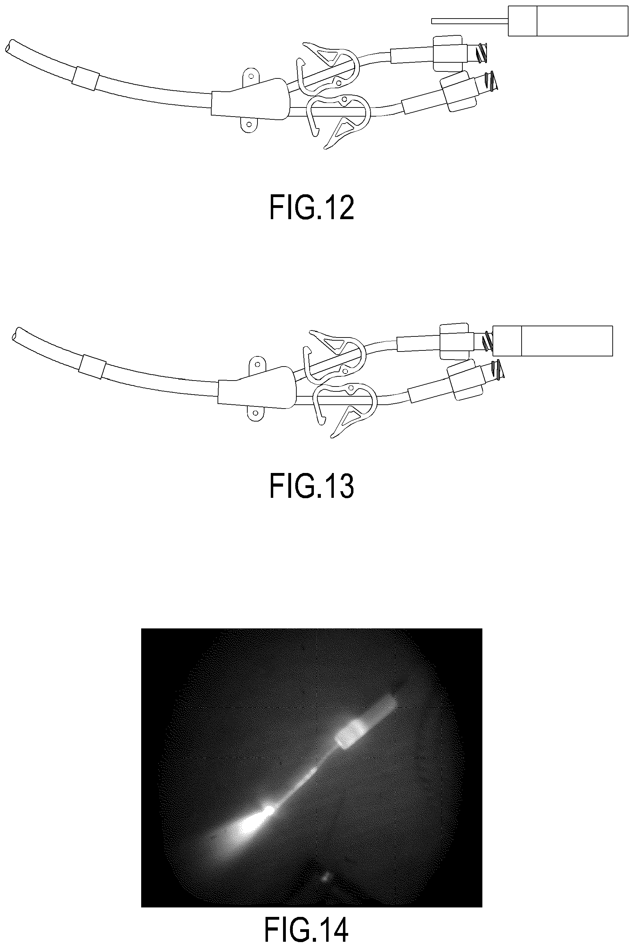

**Figures 12-13****:** Representation of the catheter cap according to the invention (Prototype) and a catheter.
**Figure 14****:** Catheter cap prototype.

### Detailed description of the invention

The present invention discloses a reusable light-activated sterilization system comprising a material having layers of graphene and/or graphene oxide, and a light emitting source of **near infrared (NIR)** radiation, preferably a light emitting diode **(LED)** source of **near infrared (NIR)** radiation, wherein the light emitting source emits radiation **in the range of 650-950 nm with an intensity of 1- 330 mW/cm².**

A material having layers of graphene and/or graphene oxide according to the present invention is a graphene-based material (GBMs) film not combined with other photo-active materials, nor functionalized with organic molecules or polymers. In the present disclosure this is also referred to as "stand-alone GBMs film". In the present disclosure this is also referred to as GBMs film.

The layers of the material having layers of graphene and/or graphene oxide according to the invention can be graphene layers or graphene oxide layers or combinations thereof. These two terms (graphene layers and graphene oxide layers) have the meaning commonly known in the field, i.e. graphene oxide is intended as the oxidized form of graphene. Preferably the graphene oxide as used herewith has a degree of oxidation higher than 10%, higher than 20%, more preferably higher than 25%, e.g. 30%.

The term "graphene material" (Gm) as used herewith refers to a single sheet of carbon atoms packed together in a crystal lattice.

The term "few-layer graphene material" (FLGm) as used herewith refers to a material having between 2 to about 5 graphene sheets, while the term "multi-layer graphene material" (MLGm) refers to stacks of between 6 to about 10 graphene sheets packed together.

The suffix "O" or "ox" is added when referring to the oxidized form (e.g. FLGOm, GOm, MLGOm).

These graphene materials can be used to form the GBMs films according to the invention, as described herewith.

In the systems according to the invention, the light-activated sterilization is exclusively due to the presence of the material having layers of graphene and/or graphene oxide, *i.e*. to the layers of graphene and/or graphene oxide combined with the irradiation of the light source as described herewith. Therefore, the sterilization system according to the invention delivers an efficient sterilization effect without the need of additional photoactive materials, or of other active components to enhance the effect.

The thickness of the material having layers of graphene and/or graphene oxide is not particularly limited, it can have a thickness of from 0.3 nm to 100 µm, preferably of from 0.5 nm to 50 µm, more preferably of from 100 nm - 45 µm.

The material having layers of graphene and/or graphene oxide according to the invention can comprise from 2 to 800 graphene or/graphene oxide layers, preferably from 2 to 200, or 2 to 100, more preferably from 2-20.

The material having layers of graphene and/or graphene oxide (stand-alone GBMs films) according to the present invention can be obtained by well-known techniques as vacuum filtration, dip-coating or spraying.

For example, the material having layers of graphene and/or graphene oxide can be obtained using vacuum filtration. This is a simple technique that allows to obtain GMBs films that can be peeled off the filtration membrane and then used as a free-standing paper, or they can be transferred onto an inert substrate. The film thickness can be controlled by adjusting the volume and concentration of the GBMs suspension (e.g. FLGm, FLGOm, MLG/MLGOm, graphene) that is vacuum filtrated, allowing the production of films ranging from few layers to up to hundreds of layers.

The GBMs suspension can be prepared using water or any other suitable organic solvents (e.g. ethanol, acetone, THF, 1-propanol, DMF, etc.). Preferably, the concentration of the GBMs suspension is from 0.5 mg/ml to 80 mg/ml, more preferably from 1 mg/ml to 50 mg/ml.

It is clear from the content of the application that the material having layers of graphene and/or graphene oxide according to the invention (GBMs film) can be a film having flexible and stable structure that can be placed/transferred onto inert substrates to facilitate the use/handling during sterilization, or it can be a film coated onto a substrate (a coating).

The coating can be obtained for example *via* dip-coating or spraying. For example, a suspension of GBMs (e.g. FLGm, FLGOm, MLGOm, graphene) is prepared and then the surface is dipped into the suspension so as to obtain a GMBs film (material having layers of graphene and/or graphene oxide) coated onto the surface. This technique can be used for depositing the material having layers of graphene and/or graphene oxide on different types of substrates without limitations.

The GBMs suspension can be prepared using water or other suitable organic solvents (ethanol, acetone, THF, 1-propanol, DMF, etc.). Preferably, the concentration of the GBMs suspension is from 0.5 mg/ml to 80 mg/ml, more preferably from 1 mg/ml to 50 mg/ml.

Examples of "material having layers of graphene and/or graphene oxide" that can be used in the present invention are graphene film, Few-layer graphene (FLG) film, Few-Layer graphene oxide (FLGO) film, Multi-Layers graphene (MLG) film, Multi-Layer graphene oxide (MLGO) film, graphite, or combinations thereof.

In one preferred embodiment, the material having layers of graphene and/or graphene oxide is a "few-layer graphene" (FLG) film.

In another preferred embodiment, the material having layers of graphene and/or graphene oxide is a "few-layer graphene oxide" (FLGO) film.

In another preferred embodiment, the material having layers of graphene and/or graphene oxide is a "few-layer graphene and few-layer graphene oxide" (FLG/FLGO) film.

**FLG/FLGO films** according to the present invention are assemblies of few-layer graphene and/or few-layer graphene oxide. This means that a FLG/FLGO film can have several layers of graphene. These layers of graphene are obtained by stacking on top of each other few-layer graphene so as to form a film or a coating that can have a thickness in the nm or µm range.

The FLG/FLGO films according to the invention are obtained starting from FLGm or FLGOm or combinations thereof. On a microscopic level therefore the FLG/FLGO films can be seen as formed by several layers of FLGm/FLGOm.

Preferably, the thickness of the FLG/FLGO films according to the invention is from 0.3 nm to 100 µm, preferably of from 0.5 nm to 50 µm, even more preferably of from 100 nm - 45 µm. The FLG/FLGO films according to the invention can comprise from 2 to 800 graphene or/graphene oxide layers, preferably from 2 to 200, or 2-100, more preferably from 2-20. The FLG/FLGO films according to the invention can be a film that can be placed/transferred onto inert substrates to facilitate the use/handling during sterilization, or they can be in the form of a coatings, i.e. a film coated onto an inert substrate (a coating).

The sterilization system according to the invention, additionally comprises a Near infrared (NIR) light emitting source, preferably a light emitting diode (LED). The Near infrared (NIR) light emitting source (e.g the light emitting diode (LED)) according to the invention emits radiation in the range of 650 - 950 nm, preferably 700 - 900 nm, preferably 750 - 850 nm, preferably 800 - 820 nm and most preferably 808 - 812 nm.

Moreover, the Near infrared (NIR) light emitting diode (LED) according to the invention emits the NIR radiation with an intensity in the range of 1-330 mW/cm², preferably of 10-250 mW/cm², more preferably of 50 - 200 mW/cm², for example 150 mW/cm². Particularly preferred stand-alone GBMs films according to the invention are FLG and FLGO films, in the present disclosure these have been shown to be very effective even when stimulated with a safe low power energy source (650nm-950nm, 1-330mW/cm²).

Accordingly, the present invention discloses a sterilization system comprising a material having layers of graphene and/or graphene oxide, also referred to as stand-alone GBMs film, wherein this material acts as NIR activated bactericidal platforms. Surprisingly this stand-alone GBMs film can deliver an antibacterial effect when used in combination with a safe low-power NIR light emitting source, preferably with light emitting diodes (LEDs).

Therefore, the sterilization systems according to the invention deliver an outstanding bactericidal action against planktonic bacteria and against adherent bacteria. Namely, almost complete elimination of bacteria in the supernatant (99%), and of adherent bacteria is obtained relying solely on the low power NIR-source irradiation of stand-alone GBMs film. The results obtained were particularly good when irradiating with a NIR-LED source according to the invention, FLG and FLGO films.

Surprisingly, the antibacterial effect of the sterilization systems disclosed herewith is against both bacteria present in the supernatant (planktonic) and adherent bacteria. This is particularly advantageous for the removal and/or prevention of biofilms that are one of the major causes of infections, especially in the medical field. Accordingly, the systems according to the invention can be used to sterilize medical devices. The sterilization systems according to the invention are particularly effective against Gram-positive bacteria.

The material having graphene and/or graphene oxide layers is irradiated with the low power NIR source (e.g. a NIR-LED source). The irradiation causes the antibacterial effect in a liquid surrounding the sterilization system and, on the surface comprising the material having graphene and/or graphene oxide layers. Therefore, the sterilization system according to the invention can be integrated, embedded, positioned in the liquid that needs sterilizing to obtain an antibacterial effect.

The positioning of the light source with respect to the material having layers of graphene and/or graphene oxide is not particularly limited. This can be placed on top of the material having layers of graphene and/or graphene oxide, or it can be placed below the material having layers of graphene and/or graphene oxide. It is also possible to have a coating of the material having layers of graphene and/or graphene oxide onto the light source.

The period of irradiation is not particularly limited, and it can be adjusted depending on the needs. For example, the surface can be irradiated for at least 1 minute, preferably for at least 10 minutes, or for at least 20 minutes, or for at least 45 minutes, or for at least 60 minutes. In one embodiment the radiation is continuously emitted from 1 to 60 minutes.

The time of irradiation, in particular, the maximum time of irradiation of the system to be sterilized is not particularly limited, and can depend on the final application. For example, the sterilization system can be used to irradiate continuously throughout the day the system to be sterilized, or it can be used to provide an intermittent irradiation repeated at regular intervals.

Without being bound to any theory, different mechanisms of action can be hypothesized for the sterilization systems according to the invention (Figure 6). Physical damage of the bacterial membrane has been mostly associated with the sharp edges of the stand-alone GBMs of the invention (e.g. FLG sharp edges), which are protruding from the surface. In addition, electron transference and charge interaction can occur due to the conductive nature and presence of oxygen-containing groups in the stand-alone GBMs (e.g. FLG and FLGO films), respectively, further contributing to the overall destabilization of the membrane. Considering the undetectable ROS production in the absence of light stimulation, physical damage associated with intrinsic ability to cause GSH oxidation seems to be powering the observed antibacterial action, supporting a ROS-independent pathway of oxidative stress.

Upon NIR-LED irradiation, even though surface chemistry and morphology may weaken the bacterial membrane, the major contribution to the overall antibacterial activity comes from the photothermal (PTT) and photodynamic (PDT) action of the graphene-based materials. The photothermal properties of material comprising graphene and/or graphene oxide layers, i.e., its ability to convert light into heat, causes surface temperature to rise to a temperature comprised from 45 °C to 60 °C, preferably comprised from 50 °C to 57 °C. For example, a surface temperature of 51.3 ºC can be obtained in the presence of FLG and a surface temperature of 56.0 ºC can be obtained upon irradiation of FLGO films. The small temperature difference between the different materials comprising graphene and/or graphene oxide according to the invention suggests that the improvement in the antibacterial performance could be mainly attributed to the photodynamic action. This is associated with the ability to induce ROS production, which combined to GSH depletion, can greatly contribute to oxidative stress - as the degree of oxidative stress relies on competing actions of ROS generation and ROS scavenging reactions.

Based on the results of DCFH-DA assay, the inventors surprisingly found out that FLG films potentiate ROS generation in a higher extent than FLGO films. Even though oxidized materials are generally associated with higher ROS production due to higher density of oxygen-containing functional groups, the conductive nature of FLG films according to the invention may allow them to act as an electron pump (causing disruption or oxidation of cellular structures without ROS production and through charge transfer from extracellular membrane to graphene). Adsorption of environmental oxygen can still occur, but in a smaller extent.

The higher depletion of GSH by the stand-alone graphene films according to the invention due to augmented oxidative potential upon irradiation, greatly contributes to enhance PDT efficiency, since GSH presence acts as ROS scavenger. This can be particularly relevant in the case of FLGO films where GSH depletion can potentiate the effect of the lower levels of ROS, while implying that a predominant ROS-independent pathway for oxidative stress is taking place. Temperature may also be playing a more active role in the mechanism of action of FLGO irradiated films, considering the lower ROS production and the greater bactericidal action. During irradiation, exposure to mild temperatures for at least 30 min may be weakening bacteria membrane structure, which associated with ROS overproduction and GSH depletion may allow excessive amounts of ROS to enter bacteria more easily, contributing to the disruption of the intracellular redox balance. Singlet oxygen (¹O₂) produced can also enhance the permeability and thermal sensitivity of the bacterial cell membrane. On the other hand, the presence of ROS can also contribute to increase the permeability of damaged bacterial membranes, making them more sensitive to heat, and thus accelerating the leakage of intracellular content from bacteria. Finally, the materials having graphene and/or graphene oxide layers according to the invention maintain their surface chemistry and morphology. Accordingly, the sterilization systems according to the invention can be used several times without degrading.

In the sterilization systems according to the invention the exposure of the material having graphene and/or graphene oxide layers to low intensity LED-NIR (650 - 950 nm with an intensity of 1 - 330 mW/cm²) drastically improve their ability to kill planktonic (up to~99%) and adherent (up to ~85%) methicillin-resistant *S*. *aureus* and *S*. *epidermidis.* Upon irradiation, a mild photothermal effect is observed in supernatant, with temperature rising from 37.0 ºC to 39.0 ºC-42.0 ºC, while surface temperature of materials having graphene layers (e.g. non-oxidized FLG films) increases to 51.3 ºC versus 56.0 ºC for materials having graphene oxide layers (e.g. FLGO films). All the materials tested prompt total glutathione oxidation when irradiated, despite FLG films induce higher ROS generation than FLGO, suggesting antioxidants depletion occurs preferentially by ROS-dependent pathway (photodynamic effect) for FLG versus ROS-independent pathway for FLGO films.

Therefore, the present invention demonstrates that safe, low-intensity NIR irradiation is a valuable and effective tool to boost graphene surfaces' antibacterial performance through a synergistic photothermal and photodynamic effect. These stand-alone NIR-activated graphene-based platforms (GBMs films) result therefore in a simple and economical disinfection surfaces/systems, with widespread use in medical and non-medical applications. These sterilization systems can therefore be applied in various healthcare applications where bacterial adhesion and infection is a concern (such as medical devices and implants), and in other non-medical applications also requiring sterilization and disinfection (such as food packaging and water treatment).

For example, the use of a NIR-LED source with an irradiation intensity of 1-330 mW/cm² renders these systems particularly useful for in vivo setting and for medical uses.

The stand-alone GMBs film according to the invention are coated onto a wave guide connected to a low power emitting source as described in the present invention, thereby resulting in an efficient integrated sterilization system that can be used to sterilize medical devices/surfaces/liquids.

The reusable light-activated sterilization system according to the invention further comprises a waveguide, in this system the waveguide is coated with said material having layers of graphene and/or graphene oxide. Preferably, waveguide is **connected to** a NIR-LED source.

For example, in one preferred embodiment the reusable light-activated sterilization system according to the invention further comprises an optical fiber, in this system the optical fiber is coated with said material having layers of graphene and/or graphene oxide. Preferably, the optic fiber is **connected to** a NIR-LED source.

This integrated system can be advantageously inserted into the liquid that needs sterilizing, or within a lumen, for example a catheter lumen, thereby resulting in an efficient integrated sterilization system and that be reused over time.

### Catheter cap

Another aspect of the present invention relates to a medical device comprising the reusable light-activated sterilization system as described in the present disclosure. In particular, the sterilization system can be integrated in a medical device in a permanent or in a removable manner. For example, the sterilization system described herewith can be integrated in a cap, thereby obtaining a sterilization cap. A preferred example of the sterilization cap according to the invention is a sterilization cap for a catheter as described herewith.

A sterilization cap for a catheter comprising:
- a cap body delimiting a compartment;
- a power source housed in said compartment;
- a light source connected to said power source for emitting near infrared (NIR) radiation in a **range of 650 nm-950nm with an intensity of 1 - 330 mW/cm²;**
- a waveguide of said NIR radiation extending from the cap body and delimiting an outer surface;
characterized in that the outer surface of said waveguide comprises a material having layers of graphene and/or graphene oxide, preferably FLG and/or FLGO.

Preferably, said waveguide is at least partially coated with the material having layers of graphene and/or graphene oxide.

In the sterilization cap according to the invention, the cap body and the waveguide can define an annular gap between each other for releasable insertion of said catheter. Moreover, the sterilization cap can comprise complementary coupling means. Preferably, said complementary coupling means are at least partially positioned at said annular gap.

Preferably, said waveguide is inserted in a tubular portion of said cap body, said tubular portion ending in said compartment on one side, and ending on the outside at an opposite second side.

The waveguide material of said waveguide is not particularly limited and can be any material that allows a NIR radiation produced by the low power emitting source (e.g by the LED source) to pass through. As an example, it could be a filament of Poly(methyl methacrylate) PMMA or a filament of Polyethylene terephthalate glycol (PETG), as well as a glass filament. This waveguide can be at least partially coated with the material having graphene and/or graphene oxide layers, these materials are as described in the present invention.

The method for partially embedding the waveguide with the material having layers of graphene and/or graphene oxide according to the invention in not particularly limited. Different methods known in the art can be used, as an example the coating of the material can be obtained through dip-coating.

For example, a suspension of GBMs (e.g. FLG, FLGO, MLGO, graphene) is prepared and then the surface is dipped into the suspension so as to obtain a GMBs film (material having layers of graphene and/or graphene oxide) onto the waveguide.

The material having layers of graphene and/or graphene oxide used in the catheter cap is as described in the present application.

The Near infrared (NIR) light emitting source, preferably the light emitting diode (LED), is as described in the present disclosure. As already discussed, this LED source emits radiation in the range of 650 - 950 nm, preferably 700 - 900 nm, preferably 750 - 850 nm, preferably 800 - 820 nm and most preferably 808 - 812 nm.

Moreover, the Near infrared (NIR) light emitting diode (LED) emits the NIR radiation with an intensity in the range of 1-330 mW/cm², preferably of 10-250 mW/cm², more preferably of 50 - 200 mW/cm², for example 150 mW/cm².

Another embodiment of the present invention relates to an assembly (KIT) comprising:
- the sterilization cap as described herewith and
- a catheter delimiting a lumen in which said waveguide of the catheter cap is at least partially insertable.

The catheter is not particularly limited, preferred types of catheter are however haemodialysis catheters, and chemotherapy catheters.

### Experimental part

In the following experimental section, the antibacterial performance of the sterilization systems according to the invention is evaluated against both planktonic and adherent bacteria. The exemplary sterilization systems are prepared using different graphene-based materials. In all the Examples, a low-power LED-based source with a peak emission of 812 nm and irradiance of 0.150 W/cm² is used. The bacterial strains used are planktonic and adherent methicillin-resistant *Staphylococcus aureus* (ATCC^{®} MRSA 33591TM) and *Staphylococcus epidermidis* (ATCC^{®} 35984TM) commercially available.

### Bacterial cultures preparation

The sterilization systems were challenged with methicillin-resistant *Staphylococcus aureus* (ATCC^{®} MRSA 33591TM) and *Staphylococcus epidermidis* (ATCC^{®} 35984TM). Bacteria were grown in Trypticase Soy Agar (TSA, Merck) plates overnight at 37 °C. Two colonies were collected, inoculated into 5 mL of Trypticase Soy Broth (TSB, Merck) and cultured overnight at 37 °C under stirring at 150 rpm. Bacterial cultures were adjusted to 10⁵ -10⁶ CFUs/mL in fresh TSB medium supplemented with 1% (v/v) human plasma, aiming to mimic the presence of proteins that naturally occurs in physiological conditions.

### Preparation of films consisting of graphene

Few-layer graphene (FLG) (~5 µm diameter, xGnP^{®} grade M) was commercially acquired from XG Sciences (Lansing, USA) and oxidized into few-layer graphene oxide (FLGO) using the modified Hummers method, as described in Henriques et al., ACS Applied Materials Interfaces 12(18) (2020) 21020-21035*.* FLG powders was dispersed in water in an ultrasonic bath for 10 min, rendering FLG aqueous suspension. Both suspensions (FLG and FLGO) (45 mg in 10 mL) were vacuum filtered through a nylon membrane (0.20 µm pore size, NY2004700, Merck) thereby obtaining FLG and FLGO films with a GBMs mass/area distribution of 7.5 mg/cm². Films were dried at 37°C overnight and cut into 9 mm diameter discs (area of 0.64 cm²) using a stainless-steel hole puncher. FLG films were slightly compressed after production, for 5 sec under ~16 kg/cm², using a Graseby Specac press, to prevent particle detachment. All films were sterilized with ethylene oxide (EO Gas series 3+). In the following examples Few-Layer graphene films and Few-layer graphene oxide films are used as graphene-based material according to the invention.

### Example 1

The FLG films prepared as reported above, were transferred to 48-well suspension plates and incubated with 300 µL of bacterial suspensions (*S.aureus* and *S*. *epidermidis)* at 37 ºC under static conditions. The remaining empty wells were filled with deionized water (dH₂O) to avoid evaporation. The samples were incubated for two hours with *S.aureus* and *S*. *epidermidis* suspensions, prepared as described above, to allow bacterial adhesion. After the two hours incubation, the samples were irradiated for 45 min with the NIR LED (812 nm, 0.150 W/cm²) at room temperature (RT). The irradiation source was placed below the well plates, i.e. the irradiation hits the plastic well first and the FLG films after. The supernatant of each sample was then collected, and surfaces rinsed two times with new TSB to remove non-adherent bacteria (washing media also recovered and added to supernatant). Viability of adherent and planktonic bacteria was analysed by live/dead fluorescent assay, and colony forming unit (CFU) and metabolic activity assays, respectively.

### Example 2

FLGO films prepared as above were transferred to 48-well suspension plates and incubated with 300 µL of bacterial suspensions (*S.aureus* and *S*. *epidermidis)* at 37 ºC under static conditions. The remaining empty wells were filled with deionized water (dH₂O) to avoid evaporation. The FLGO films were incubated for two hours with *S.aureus* and *S*. *epidermidis* suspensions to allow bacterial adhesion. After the two hours incubation, the samples were irradiated for 45 min with the NIR LED (812 nm, 0.150 W/cm²) at room temperature (RT). The irradiation source was placed below the well plates, i.e. the irradiation hits the plastic well first and the FLGO films after. The supernatant of each sample was then collected, and surfaces rinsed two times with new TSB to remove non-adherent bacteria (washing media also recovered and added to supernatant). Viability of adherent and planktonic bacteria was analysed by live/dead fluorescent assay, and colony forming unit (CFU) and metabolic activity assays, as described below.

### Comparative Example 1

The samples were prepared in the same way as in Example 1, except that the samples were not irradiated. The 45 min waiting time is also maintained (nor irradiation during this time).

### Comparative Example 2

The samples were prepared in the same way as in Example 2, except that the samples were not irradiated. The 45 min waiting time is also maintained (nor irradiation during this time).

### Comparative Example 3

Silicone films, used as comparative example, were transferred to 48-well suspension plates and incubated with 300 µL of bacterial suspensions (*S.aureus* and *S*. *epidermidis)* at 37 ºC under static conditions. The remaining empty wells were filled with deionized water (dH₂O) to avoid evaporation. The silicone films were incubated for two hours with *S.aureus* and *S*. *epidermidis* suspensions to allow bacterial adhesion. After the two hours incubation, the samples were irradiated for 45 min with the NIR LED (812 nm, 0.150 W/cm²) at room temperature (RT). The irradiation source was placed below the well plates, i.e. the irradiation hits the plastic well first and the silicone films after. The supernatant of each sample was then collected, and surfaces rinsed two times with new TSB to remove non-adherent bacteria (washing media also recovered and added to supernatant). Viability of adherent and planktonic bacteria was analysed by live/dead fluorescent assay, and colony forming unit (CFU) and metabolic activity assays, as described below.

### NIR irradiation of FLG and FLGO films

FLG and FLGO films were either kept in the dark (NIR off) (Comparative Examples 1-2) or exposed to NIR light (NIR on) (Examples 1-2, and Comparative Example 3). A low-power LED-based source with peak emission of 812 nm and irradiance of 0.150 W/cm² was used to irradiate the films. The irradiation source was placed below the well plates, i.e. the irradiation hits the plastic well first and the FLG films after. In order to expose bacteria for at least 30 min to the maximum temperature, 45 min was selected as the irradiation time for the antibacterial assays. The antibacterial activity was thereby explored.

### Characterization methods

The supernatant of each sample (examples above) was collected, and surfaces rinsed two times with new TSB to remove non-adherent bacteria (washing media was also recovered and added to supernatant). Both supernatant and surface of the films were analysed to assess planktonic and adherent bacteria, respectively as described below.

### Planktonic bacteria

Viability of planktonic bacteria was evaluated by colony forming units using the agar plate culture method. For that, serial dilutions (10⁻¹, 10⁻², 10⁻³) of the supernatants were prepared and three drops of 10 µL of each dilution plated in TSA. Plates were incubated overnight at 37 ºC and CFUs counted. Bacteria viability was also confirmed by alamarBlue^{™} assay. For that, resazurin was added to supernatants (10% v/v) and incubated for 2 h at 37 ºC. RFUs were measured (λₑₓ/λₑₘ: 530 nm/590 nm) in a microplate fluorometer (Spectra Max GeminiXS, Molecular Devices, California, USA) and correlated with the metabolic activity of bacteria present in the medium. Eight replicates of each sample (Examples and Comparative examples) were evaluated.

### Adherent bacteria

Viability of adherent bacteria was determined by fluorescence staining using the LIVE/DEAD^{®} Baclight^{™} Bacterial Viability Kit (L13152, Invitrogen). A mixture of SYTO9 (λₑₓ/λₑₘ = 480/500 nm, green fluorescence) and propidium iodide (PI) (λₑₓ/λₑₘ = 490/635 nm, red fluorescence) nucleic acid stains was incubated with samples for 15 min in the dark at RT. Samples were transferred to uncoated 24-well µ-plates (#82406, IBIDI, Germany) with surface facing the glass bottom of the plate. Fluorescence images were acquired using a high-content screening microscope IN Cell Analyzer 2000 (GE Healthcare, Nikon 40×/0.95 NA Plan Apo objective) using a CCD Camera (CoolSNAP K4). Three replicates of each sample were evaluated, acquiring 9 fields per sample (each field across a z-section of ~25 µm, with a z-step of 1 µm), with a total spanning area of 1.44 mm². Z-sections were projected on one plane using the IN Cell Investigator Developer Toolbox v.1.9.2 (GE Healthcare). Bacteria quantification was performed as previously described in Henriques et al., ACS Applied Materials Interfaces 12(18) (2020) 21020-21035*.*

### Impact of NIR irradiation in surface physicochemical features

NIR radiation impact on surface morphology and chemistry (elemental composition and oxidation degree) of films was evaluated by Scanning Electron Microscopy (SEM) and X-ray Photoelectron Spectroscopy (XPS), respectively. FLG/FLGO films were continuously irradiated for 45 min (812 nm, 0.150 W/cm²).

### Scanning Electron Microscopy (SEM)

SEM analysis was performed using a Phenom XL SEM in a Secondary Electron Detector (SED) mode and with an acceleration voltage of 10 kV. FLG and FLGO films were adhered onto carbon tape and coated with a thin layer of Au by sputtering to improve their conductivity.

### X-ray Photoelectron Spectroscopy (XPS)

XPS analysis was conducted using a Kratos Axis Ultra HSA (Kratos Analytical, U.K.) equipment with a monochromatic Al X-ray source operating at 15 kV (90 W). Survey spectra were acquired at 80 eV and C 1s and O 1s high-resolution spectra at 40 eV, setting the reference of the C 1s peak to 284.6 eV. Deconvolution of high-resolution spectra was performed with CasaXPS processing software version 2.3.16, using background type Shirley. C 1s deconvolution was performed as previously described in Henriques et al., ACS Applied Materials Interfaces 12(18) (2020) 21020-21035*.*

### Impact of NIR irradiation in photothermal properties

The light-to-heat conversion capacity of GBMs films upon NIR irradiation was also investigated. FLG/FLGO films were transferred to a 48-well suspension plate, immersed in 300 µL of TSB, and incubated for 2 h at 37 °C. After that, samples were exposed to NIR light (812 nm, 0.150 W/cm²), and the temperature of both the surrounding medium and the surface of the films was recorded each 15 min, during 60 min, using a type K thermocouple microprobe. Three replicates were used per condition and two independent experiments were conducted. Silicone films were used as a control surface.

### Impact of NIR irradiation in photodynamic properties

Glutathione (GSH) levels, a key endogenous antioxidant, and reactive oxygen species (ROS) generation, are two measures of oxidative stress in biological systems. Both can contribute to the photodynamic effect and therefore were quantified.

### GSH oxidation assay

To determine GSH levels, GBMs films were incubated with 300 µL of GSH (1.5 mM, Sigma) in reaction buffer (0.1 M sodium bicarbonate (pH 8), containing 1 mM EDTA) for 2 h at 37 °C. After incubation, samples were either kept in the dark (NIR off) or NIR irradiated (NIR on) for 45 min (812 nm, 0.150 W/cm2). After that, 50 µL of the supernatant was mixed with 500 µL of the reaction buffer and 10 µL of Ellman's reagent (10 mM in reaction buffer) for 15 min in the dark, allowing its reaction with the thiol groups of GSH to yield fluorescent TNB. GSH was quantified spectrophotometrically at 412 nm and the loss of GSH was calculated. GSH in reaction buffer without GBMs films was used as negative control, whereas GSH in H₂O₂ (1 mM) was used as positive control of oxidation.

### DCFH-DA assay

To assess the generation of ROS, GBMs films were incubated alone or with bacteria for 2 h at 37 ºC, followed by addition of 2',7'-dichlorodihydrofluorescein diacetate (DCFH-DA, ThermoFisher) at 10 µM. Samples were either kept in the dark (NIR off) or NIR irradiated (NIR on) for 45 min (812 nm, 0.150 W/cm²). Fluorescence intensities were measured at 527 nm with excitation at 495 nm in a microplate fluorometer (Spectra Max GeminiXS, Molecular Devices, California, USA). Bacteria exposed to radiation were considered as basal activity and subtracted to the fluorescence intensities of films + bacteria.

### Results

Antibacterial performance of FLG and FLGO films was evaluated towards planktonic and adherent methicillin-resistant *S*. *aureus* and *S*. *epidermidis* after irradiation with a safe, low power (0.150 W/cm²) near-infrared (NIR) (812 nm) diode (Examples 1 and 2). Comparing to non-irradiated samples (NIR off) (Comparative Examples 1 and 2), viability of planktonic bacteria was drastically reduced after 45 min irradiation (NIR on). Almost 90% of bacteria were eliminated when in the presence of FLG films and close to 99% with FLGO films (reaching a 2log reduction) (Figure 1), independently of the bacterial strain. Results were consistent in both techniques used to evaluate bacteria viability, namely CFU counting and metabolic activity. Furthermore, in silicone (Comparative Example 3), used as control irradiated material, no bacterial death was observed, demonstrating that the antibacterial action is induced by the LED-NIR irradiated GBMs film. Furthermore, control bacterial culture showed no reduction of viability upon irradiation, indicating that LED-NIR irradiation alone was harmless to both bacterial strains.

Figure 1 shows the antibacterial effect of the sterilization systems of Examples 1 and 2 (FLG and FLGO films) on planktonic bacteria. After 2 h incubation with *S*. *aureus* and *S*. *epidermidis,* FLG and FLGO films were either kept in the dark (NIR off) (Comparative Examples 1 and 2) or irradiated (NIR on) for 45 min (812 nm, 0.150 W/cm²) (Examples 1 and 2), the sample were prepared as described in the Examples above. Bacteria from the supernatant were evaluated by CFU counting and bacterial metabolic activity measurement. Silicone films (Comparative Example 3) were used as a control material. Bacteria cultured in the absence of any material (Bact) was used as the control of the irradiation effect on the bacteria viability. *statistical significance, p < 0.05: one-way ANOVA test to compare films within the same condition (either NIR off or NIR on), and unpaired t-test with Welch's correction to compare the same film between NIR off or NIR on conditions; *alone indicates significant differences from its counterpart in NIR.

As a result of NIR irradiation (NIR on), adherent bacteria in Examples 1 and 2 also showed an up to 80% increase in death percentage, and a significant reduction in adhesion, particularly when in the presence of FLGO films (Example 2) and towards methicillin-resistant *S*. *aureus* (Figure 2, and Figures 7 and 8 for fluorescent microscopy images). In the absence of irradiation (NIR off), (Comparative Examples 1-2) methicillin-resistant *S*. *aureus* was already more susceptible to the action of FLG and FLGO films, with *S*. *epidermidis* showing a more pronounced death increase upon irradiation (NIR on). This is denoted by the increased uptake of propidium iodide (PI) dye that only enters bacteria with compromised membrane. No differences were seen in silicone surfaces before and after irradiation (Comparative Example 3), validating that the observed bactericidal effect of the sterilization systems according to the invention.

Figure 2 shows the antibacterial effect of the sterilization systems of Examples 1 and 2 (FLG and FLGO films) against adherent bacteria. After 2 h incubation with *S*. *aureus* and *S*. *epidermidis,* FLG and FLGO films were either kept in the dark (NIR off) (Comparative Examples 1 and 2) or irradiated (NIR on) for 45 min (812 nm, 0.150 W/cm²) (Examples 1 and 2). Adherent bacteria were stained with SYTO9 (live) or PI (dead), visualized by fluorescent microscopy (Figure 7 and Figure 8), and counted.

### Mechanism of action

### Surface physicochemical features

Figure 3 shows the physicochemical features of FLG and FLGO films. (A) is the surface morphology and (B) is the chemistry of films when kept in the dark (NIR off) and when irradiated (NIR on) for 45 min (812 nm, 0.150 W/cm²). Exposure to NIR irradiation for 45 min caused no alterations in neither surface morphology (Figure 3.A) nor chemistry (Figure 3.B) of the FLG and FLGO films. Surface features were similar in both conditions (NIR off and NIR on), with sharp edges protruding in FLG films and wrinkles with similar morphology and size being observed in FLGO films. Oxidized materials presented high amounts of oxygen (~30%) and maintenance of the overall elemental composition upon irradiation, with a carbon/oxygen (C/O) ratio of 28.4 (NIR off) vs 26.75 (NIR on) for FLG films, and 2.17 (NIR off) vs 2.23 (NIR on) for FLGO films. The percentages of chemical groups, and in particular oxygen-containing groups (hydroxyl groups (C-OH), epoxy groups (C-O-C), carbonyl groups (C=O), carboxyl groups (O-C=O)), were also similar (Figure 9), confirming that neither oxidation nor reduction was induced by LED-NIR radiation. In the absence of irradiation, surface morphology and chemistry seem to contribute to the inherent bactericidal or anti-adhesive activity of FLG and FLGO films. However, upon exposure to NIR radiation, something else than just these features *per se* is driving the overall mechanism of action.

### Photothermal effect

The photothermal effect relies on a local increase in the temperature to kill bacteria. Photothermal properties of FLG and FLGO films were assessed in conditions similar to the antibacterial assays (after 2h incubation in culture media at 37 ºC) through evaluation of the temperature of the supernatant and surface of samples over 60 min of irradiation (Figure 4). In the presence of both FLG and FLGO films, temperature of the supernatant increased during the first 30 min, remaining similar afterwards and within the suitable range for bacterial growth and viability (between 33 ºC to 41 ºC). Surface temperature, however, showed a steep increase during the first 15 min, attaining 50 ºC in FLG and 55 ºC in FLGO films. After 45 min irradiation, temperatures reached 51.3 ºC and 56 ºC in FLG and FLGO films, respectively, allowing adherent bacteria to be subjected to high temperatures for at least 30 min, thus potentiating the photothermal-induced bactericidal effect. In the absence of GBMs films, temperature of the culture media (TSB) stayed below 37 ºC, similarly to when silicone films were irradiated. This shows that LED-NIR alone has no capacity to induce an increase in temperature, proving the excellent photothermal efficiency derived from GBMs films, even with low intensity radiation.

### Photodynamic effect

The photodynamic effect relies on ROS production to kill bacteria. This boost in ROS production disrupts the balance between ROS generation and elimination, leading to oxidative stress. GSH, an endogenous antioxidant, is crucial to maintain this balance; its elimination potentiates the photodynamic effect by failing to eliminate ROS. The intrinsic oxidative potential of FLG and FLGO films caused a GSH oxidation of around 75% (NIR off). Exposure to NIR irradiation (NIR on) prompted a significant increase in GSH oxidation to 95% (Figure 5.A), as denoted by a gradual paling of its typical yellow color, due to reaction between GSH and Ellman's reagent, quantitatively translated into a decrease in the absorbance at 412 nm. Silicone surfaces showed negligible oxidative potential, independently of the irradiation by NIR light.

Oxygen-containing groups on FLGO films can potentiate GSH oxidation, considering that more reactive defect sites are available to adsorb O₂ and water molecules, which increases surface oxides and hydroxyl radicals formation. Apart from having reactive points, FLG films conductive nature may also be stimulating GSH oxidation, with FLG acting as an electron acceptor.

Agents with photodynamic action potentiate ROS production. In the absence of NIR stimulation (NIR off), all graphene-based films elicited negligible amounts of DCF fluorescence, suggesting the lack of ROS in the presence of both GBM films and GBM films with bacteria (Figure 5.B). Exposure of films to NIR irradiation (NIR on) caused a significant increase in DCF fluorescence, indicating a NIR-triggered ROS generation as a consequence of the association between the GBMs films and NIR radiation. Silicone films showed residual levels of ROS production, independently of the condition. When the GBM films are in the presence of bacteria, ROS levels are maintained independently of the strain used. This suggests that either bacteria are managing/balancing out ROS overproduction, which is unlikely considering their high death percentage, or ROS molecules are interacting with the bacterial membrane, leading to ROS elimination and bacterial damage or death. Based on both assays, an explanation for what is happening can be hypothesized (Figure 5.C). In the absence of NIR (NIR off), FLG and FLGO films actively contribute to GSH oxidation, however they did not mediate the generation of ROS. On the other hand, exposure to radiation (NIR on) augmented FLG and FLGO capacity to oxidize GSH (represented as thicker arrows) while also inducing ROS production. Despite with similar GSH oxidation, FLG showed significantly higher capacity to induce ROS production than FLGO, thus suggesting ROS-mediated killing as the dominant factor in the antibacterial activity of FLG films. The lower ability to induce ROS production indicates that FLGO films may act primarily through a ROS-independent pathway. Combination of both GSH oxidation and ROS production potentiates oxidative stress, due to inability of ROS neutralization and consequent accumulation. This increases the efficacy of the photodynamic effect, leading to collapse of cellular functions through the damage of DNA and vital enzymes and bacterial death.

## Claims

1. Reusable light-activated sterilization system comprising:
- a material having layers of graphene and/or graphene oxide,
- a light emitting source of **near infrared (NIR)** radiation, and
- a waveguide,
wherein the light emitting source emits radiation **in the range of 650 - 950 nm with an intensity of 1** - **330 mW/cm²,** and wherein said waveguide is coated with the material having layers of graphene and/or graphene oxide.

2. The reusable light-activated sterilization system according to claim 1, wherein the light emitting source of near infrared (NIR) radiation is a light emitting diode **(LED).**

3. The reusable light-activated sterilization system according to claim 1 or 2, wherein the light-activated sterilization is due to the presence of the layers of graphene and/or graphene oxide.

4. The reusable light-activated sterilization system according to any of claims 1-3, wherein the material having layers of graphene and/or graphene oxide has up to 800 layers of graphene and/or graphene oxide.

5. The reusable light-activated sterilization system according to claim 1, wherein the said waveguide is **connected to** the light emitting source.

6. The reusable light-activated sterilization system according to any of claims 1-4, wherein:
the light emitting source emits radiation in the range of **750-850 nm;** and/or
the radiation is continuously emitted for 1-60 minutes.

7. The reusable light-activated sterilization system according to any of claims 1-6, wherein the light emitting source emits radiation **with an intensity of 10-250 mW/cm².**

8. The reusable light-activated sterilization system according to any of claims 1-7, wherein the material having layers of graphene and/or graphene oxide is:
a few-layer graphene film; or
a few-layer graphene oxide film.

9. The reusable light-activated sterilization system according to any of claims 1-7, wherein the material having layers of graphene and/or graphene oxide is a multi-layer graphene film and/or a multilayer-layer oxide film.

10. The reusable light-activated sterilization system according to any of claims 1-9, wherein material having layers of graphene and/or graphene oxide has a thickness of 0.3 nm to 100 µm.

11. A medical device comprising the reusable light-activated sterilization system according to any of claims 1-10, optionally, wherein the medical device is:
a catheter cap; or
a catheter, preferably a haemodialysis catheter or a chemotherapy catheter.

12. A sterilization cap for a catheter comprising:
- a cap body delimiting a compartment;
- a power source housed in said compartment;
- a light source connected to said power source for emitting near infrared (NIR) radiation in a **range of 650 nm-950nm with an intensity of 1 - 330 mW/cm²;**
- a waveguide of said NIR radiation extending from the cap body and delimiting an outer surface;
**characterized in that** the outer surface of said waveguide comprises a material having layers of graphene and/or graphene oxide.

13. The catheter cap according to claim 12, wherein said waveguide is at least partially coated with the material having layers of graphene and/or graphene oxide.

14. The catheter cap according to any of claims 12-13, wherein the material having layers of graphene and/or graphene oxide has up to 800 layers of graphene and/or graphene oxide.

15. The catheter cap according to any of claims 12-14, wherein the material having layers of graphene and/or graphene oxide is a few-layer graphene film or a few-layer graphene oxide film.

## Patentansprüche

1. Wiederverwendbares, lichtaktiviertes Sterilisationssystem, umfassend:
- ein Material, das Schichten aus Graphen und/oder Graphenoxid aufweist,
- eine lichtemittierende Quelle für **Nahinfrarotstrahlung (NIR),** und
- einen Wellenleiter, wobei die lichtemittierende Quelle Strahlung **im Bereich von 650 bis 950 nm mit einer Intensität von 1 bis 330 mW/cm²** emittiert, und wobei der Wellenleiter mit dem Material beschichtet ist, das Schichten aus Graphen und/oder Graphenoxid aufweist.

2. Wiederverwendbares, lichtaktiviertes Sterilisationssystem nach Anspruch 1, wobei die lichtemittierende Quelle für Nahinfrarotstrahlung (NIR) eine Leuchtdiode **(LED)** ist.

3. Wiederverwendbares, lichtaktiviertes Sterilisationssystem nach Anspruch 1 oder 2, wobei die lichtaktivierte Sterilisation auf das Vorhandensein der Schichten aus Graphen und/oder Graphenoxid zurückzuführen ist.

4. Wiederverwendbares, lichtaktiviertes Sterilisationssystem nach einem der Ansprüche 1 bis 3, wobei das Material mit Schichten aus Graphen und/oder Graphenoxid bis zu 800 Schichten aus Graphen und/oder Graphenoxid aufweist.

5. Wiederverwendbares, lichtaktiviertes Sterilisationssystem nach Anspruch 1, wobei der Wellenleiter mit der lichtemittierenden Quelle **verbunden** ist.

6. Wiederverwendbares, lichtaktiviertes Sterilisationssystem nach einem der Ansprüche 1 bis 4, wobei:
die lichtemittierende Quelle Strahlung im Bereich von **750 bis 850 nm** emittiert; und/oder
die Strahlung kontinuierlich für 1-60 Minuten emittiert wird.

7. Wiederverwendbares, lichtaktiviertes Sterilisationssystem nach einem der Ansprüche 1 bis 6, wobei die lichtemittierende Quelle Strahlung **mit einer Intensität von 10 bis 250 mW/cm²** emittiert.

8. Wiederverwendbares, lichtaktiviertes Sterilisationssystem nach einem der Ansprüche 1 bis 7, wobei das Material, das Schichten aus Graphen und/oder Graphenoxid aufweist, Folgendes ist:
ein Graphenfilm mit wenigen Schichten; oder
ein Graphenoxidfilm mit wenigen Schichten.

9. Wiederverwendbares, lichtaktiviertes Sterilisationssystem nach einem der Ansprüche 1 bis 7, wobei das Material, das Schichten aus Graphen und/oder Graphenoxid aufweist, ein Graphenfilm mit mehreren Schichten und/oder ein Oxidfilm mit mehreren Schichten ist.

10. Wiederverwendbares lichtaktiviertes Sterilisationssystem nach einem der Ansprüche 1 bis 9, wobei das Material, das Schichten aus Graphen und/oder Graphenoxid aufweist, eine Dicke von 0,3 nm bis 100 µm aufweist.

11. Medizinische Vorrichtung, umfassend das wiederverwendbare, lichtaktivierte Sterilisationssystem nach einem der Ansprüche 1 bis 10, wobei die medizinische Vorrichtung optional Folgendes ist:
eine Katheterkappe; oder
ein Katheter, vorzugsweise ein Hämodialysekatheter oder ein Chemotherapiekatheter.

12. Sterilisationskappe für einen Katheter, umfassend:
- einen Kappenkörper, der ein Fach abgrenzt;
- eine Energiequelle, die in diesem Fach untergebracht ist;
- eine mit der Energiequelle verbundene Lichtquelle zur Emission von Nahinfrarotstrahlung (NIR) **in einem Bereich von 650 bis 950 nm mit einer Intensität von 1 bis 330 mW/cm²;**
- ein Wellenleiter für die NIR-Strahlung, der sich von dem Kappenkörper aus erstreckt und eine äußere Oberfläche abgrenzt;
**dadurch gekennzeichnet, dass** die äußere Oberfläche des Wellenleiters ein Material umfasst, das Schichten aus Graphen und/oder Graphenoxid aufweist.

13. Katheterkappe nach Anspruch 12, wobei der Wellenleiter mindestens teilweise mit dem Material beschichtet ist, das Schichten aus Graphen und/oder Graphenoxid aufweist.

14. Katheterkappe nach einem der Ansprüche 12 bis 13, wobei das Material, das Schichten aus Graphen und/oder Graphenoxid aufweist, bis zu 800 Schichten aus Graphen und/oder Graphenoxid aufweist.

15. Katheterkappe nach einem der Ansprüche 12 bis 14, wobei das Material, das Schichten aus Graphen und/oder Graphenoxid aufweist, ein Graphenfilm mit wenigen Schichten oder ein Graphenoxidfilm mit wenigen Schichten ist.

## Revendications

1. Système de stérilisation activé par la lumière réutilisable comprenant :
- un matériau ayant des couches de graphène et/ou d'oxyde de graphène,
- une source émettrice de lumière de rayonnement **proche infrarouge (NIR),** et
- un guide d'ondes,
dans lequel la source émettrice de lumière émet un rayonnement **dans la plage de 650 - 950 nm avec une intensité de 1** - **330 mW/cm²**, et dans lequel ledit guide d'ondes est revêtu avec le matériau ayant des couches de graphène et/ou d'oxyde de graphène.

2. Système de stérilisation activé par la lumière réutilisable selon la revendication 1, dans lequel la source émettrice de lumière de rayonnement proche infrarouge (NIR) est une diode électroluminescente **(DEL).**

3. Système de stérilisation activé par la lumière réutilisable selon la revendication 1 ou la revendication 2, dans lequel la stérilisation activée par la lumière est due à la présence des couches de graphène et/ou d'oxyde de graphène.

4. Système de stérilisation activé par la lumière réutilisable selon l'une quelconque des revendications 1 à 3, dans lequel le matériau ayant des couches de graphène et/ou d'oxyde de graphène a jusqu'à 800 couches de graphène et/ou d'oxyde de graphène.

5. Système de stérilisation activé par la lumière réutilisable selon la revendication 1, dans lequel ledit guide d'ondes est **connecté à** la source émettrice de lumière.

6. Système de stérilisation activé par la lumière réutilisable selon l'une quelconque des revendications 1 à 4, dans lequel :
la source émettrice de lumière émet un rayonnement dans la plage de **750 - 850 nm** ; et/ou
le rayonnement est émis en continu pendant 1 - 60 minutes.

7. Système de stérilisation activé par la lumière réutilisable selon l'une quelconque des revendications 1 à 6, dans lequel la source émettrice de lumière émet un rayonnement **d'une intensité de 10** - **250 mW/cm².**

8. Système de stérilisation activé par la lumière réutilisable selon l'une quelconque des revendications 1 à 7, dans lequel le matériau ayant des couches de graphène et/ou d'oxyde de graphène est :
un film de graphène à quelques couches ; ou
un film d'oxyde de graphène à quelques couches.

9. Système de stérilisation activé par la lumière réutilisable selon l'une quelconque des revendications 1 à 7, dans lequel le matériau ayant des couches de graphène et/ou d'oxyde de graphène est un film de graphène multicouches et/ou un film d'oxyde multicouches.

10. Système de stérilisation activé par la lumière réutilisable selon l'une quelconque des revendications 1 à 9, dans lequel un matériau ayant des couches de graphène et/ou d'oxyde de graphène a une épaisseur de 0,3 nm à 100 µm.

11. Dispositif médical comprenant le système de stérilisation activé par la lumière réutilisable selon l'une quelconque des revendications 1 à 10, facultativement, dans lequel le dispositif médical est :
un capuchon de cathéter ; ou
un cathéter, de préférence un cathéter d'hémodialyse ou un cathéter de chimiothérapie.

12. Capuchon de stérilisation pour un cathéter comprenant :
- un corps de capuchon délimitant un compartiment ;
- une source d'alimentation logée dans ledit compartiment ;
- une source de lumière connectée à ladite source d'alimentation pour émettre un rayonnement proche infrarouge (NIR) dans une **plage de 650 nm - 950 nm avec une intensité de 1** - **330 mW/cm²** ;
- un guide d'ondes dudit rayonnement proche infrarouge s'étendant à partir du corps du capuchon et délimitant une surface externe ;
**caractérisé en ce que** la surface externe dudit guide d'ondes comprend un matériau ayant des couches de graphène et/ou d'oxyde de graphène.

13. Capuchon de cathéter selon la revendication 12, dans lequel ledit guide d'ondes est au moins partiellement revêtu du matériau ayant des couches de graphène et/ou d'oxyde de graphène.

14. Capuchon de cathéter selon l'une quelconque des revendications 12 à 13, dans lequel le matériau ayant des couches de graphène et/ou d'oxyde de graphène a jusqu'à 800 couches de graphène et/ou d'oxyde de graphène.

15. Capuchon de cathéter selon l'une quelconque des revendications 12 à 14, dans lequel le matériau ayant des couches de graphène et/ou d'oxyde de graphène est un film de graphène à quelques couches ou un film d'oxyde de graphène à quelques couches.
